(19) **Europäisches Patentamt European Patent Office Office européen des brevets**

(11) **EP 3 738 621 B1**

(12) **EUROPEAN PATENT SPECIFICATION**

(45) Date of publication and mention of the grant of the patent:
**08.10.2025  Bulletin 2025/41**

(21) Application number: **19738380.5**

(22) Date of filing: **10.01.2019**

(51) International Patent Classification (IPC):
**A61L 27/24** (2006.01)     **A61L 27/18** (2006.01)
**A61L 27/22** (2006.01)     **A61L 27/54** (2006.01)
**A61L 27/58** (2006.01)

(52) Cooperative Patent Classification (CPC):
(C-Sets available)
**A61L 27/18; A61L 27/22; A61L 27/54; A61L 27/58;**
A61L 2300/25; A61L 2300/434; A61L 2300/622;
A61L 2400/06; A61L 2430/34          (Cont.)

(86) International application number:
**PCT/KR2019/000404**

(87) International publication number:
**WO 2019/139381 (18.07.2019 Gazette 2019/29)**

(54) **COLLAGEN PEPTIDE-CONTAINING POLYCAPROLACTONE MICROSPHERE FILLER AND PREPARATION METHOD THEREFOR**

KOLLAGENPEPTIDHALTIGER POLYCAPROLACTONMIKROKUGELFÜLLSTOFF UND HERSTELLUNGSVERFAHREN DAFÜR

CHARGE DE MICROSPHÈRES DE POLYCAPROLACTONE CONTENANT UN PEPTIDE DE COLLAGÈNE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **10.01.2018   KR 20180003585**

(43) Date of publication of application:
**18.11.2020   Bulletin 2020/47**

(73) Proprietor: **G2Gbio, Inc.**
**Daejeon 34054 (KR)**

(72) Inventors:
• **LEE, Heeyong**
  **Daejeon 34032 (KR)**
• **SEOL, Eunyoung**
  **Daejeon 34080 (KR)**
• **YOON, Kwonhyeok**
  **Daejeon 34046 (KR)**
• **NA, Yongha**
  **Daejeon 34046 (KR)**

(74) Representative: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

(56) References cited:
**EP-A1- 2 803 351          WO-A1-00/15188**
**WO-A1-2005/097061     WO-A2-2009/014441**
**KR-A- 20100 065 309    KR-A- 20120 091 982**
**KR-A- 20140 135 337    KR-A- 20170 123 099**
**KR-B1- 101 685 312      US-B2- 6 531 160**

• **DATABASE WPI Week 201757, Derwent World Patents Index; AN 2017-493577, XP002804108**
• **DATABASE WPI Week 201707, Derwent World Patents Index; AN 2016-81204Q, XP002804109**
• **DATABASE WPI Week 201803, Derwent World Patents Index; AN 2017-782200, XP002804110**

(52) Cooperative Patent Classification (CPC): (Cont.)

C-Sets
**A61L 27/18, C08L 67/04**

**Description**

**[Technical Field]**

The present disclosure relates to a polycaprolactone microsphere filler and a method for preparing the same, and more particularly, to a polycaprolactone microsphere filler which not only has resolved the in vivo stability problems of collagen peptide by containing collagen peptide therein, but also when applied to a living body, rapidly exhibits effects after treatment, and further maintains the effects for a long period of time, and a method for preparing the same.

**[Background Art]**

Dermal fillers are an injection type medical instrument that injects materials safe for a human body into a dermic layer of face to replenish dermal tissues, such as improving wrinkles and volume in aesthetic appearance, and are used for so-called anti-aging treatments, including botulinum toxin (BOTOX®), autologous fat transplantation, thread lift, microneedle, laser treatment, dermabrasion, and the like.

The first-generation dermal filler that was first developed is an animal-derived collagen filler, and is rarely used in recent years because the duration of effect after treatment is short from 2 to 4 months and it is troublesome that dermal hypersensitivity reaction tests must be performed one month before treatment.

The second-generation dermal filler is a hyaluronic acid filler, and is currently the most frequently used filler in that it has a longer duration of effect than a collagen filler, and has significantly less side effects such as dermal hypersensitivity reactions because it is composed of polysaccharides similar to the components of the human body and thus do not require dermal reaction tests like collagen fillers. In particular, hyaluronic acid is easy to treat and remove, has excellent viscoelasticity, maintains the moisture, volume and elasticity of the skin, which is thus very suitable as a raw material for dermal fillers. Recently, studies have been actively conducted to extend the duration of effect by inducing cross-linking of hyaluronic acid to increase particle size and molecular weight, but since the duration of effect is relatively short from 6 to 12 months, it is troublesome that the treatments must be repeated every 6 to 12 months.

The third-generation dermal filler, which is a synthetic polymer filler such as polylactic acid (PLA) or polycaprolactone (PCL), is very gradually decomposed in the human body, and therefore, is used for the purpose of exhibiting a longer duration of effect as compared with collagen and hyaluronic acid fillers which are absorbent fillers. In particular, polycaprolactone is 100% absorbed by the human body and thus is a safe component, and is advantageous in that it is absorbed more slowly than polylactic acid after being implanted into the skin, promotes the production of collagen, and lasts the effect for 1 to 4 years as a soft-feeling tissue having no foreign matter feeling. However, the polycaprolactone filler is a filler in the form of a microsphere and must be administered by suspending it in a gel carrier such as carboxymethylcellulose (CMC), and shows the effects only after 6-8 weeks after injection into the skin. Thus, this is disadvantageous in that the satisfaction of the treatment is lower than that of the hyaluronic acid filler showing an immediate effect after the treatment.

Meanwhile, it is known that bioactive peptides, such as KTTKS, are collagen-derived substances, which inhibit the synthesis of collagenase, or promote the production of extracellular matrix (ECM), and promote the expression of type I and type III collagen and fibronectin. However, it is a fact that due to the low in vivo stability of peptides and low skin permeability, the bioactive peptides are used restrictively to cosmetics and the like for the purpose of improving wrinkles and regenerating skin by utilizing various derivatives.

Therefore, there is a need to develop a new polycaprolactone microparticle filler with improved efficacy by solving the in vivo stability problem of collagen peptide by utilizing the properties of the existing polycaprolactone microparticle filler and encapsulating collagen peptide with polycaprolactone microspheres.

The WO 2005/097061 A1 discloses compositions comprising encapsulated peptide copper complexes, and, additionally, to such compositions formulated for use as pharmaceutical and cosmetic products, as well as to medical devices that comprise such compositions.

The U S6,531,160 B2 discloses microcapsules with an aqueous core containing at least one water-soluble cosmetic or dermatological active principle, and with a polymeric and/or waxy envelope, in which the said envelope consists of at least one polymer chosen from polycaprolactone, poly(3-hydroxybutyrate), poly(ethylene adipate), poly(butylene adipate), cellulose esters of at least one C1-C4 carboxylic acid, copolymers of styrene and of maleic anhydride, copolymers of styrene and of acrylic acid, styrene-ethylene/butylene-styrene block terpolymers, styrene-ethylene/propylene-styrene block terpolymers and terpolymers of ethylene, of vinyl acetate and of maleic anhydride, and/or of at least one wax chosen from beeswax, polyglycerolated beeswax, hydrogenated plant oils, paraffin with a melting point above 45° C., and silicone waxes.

The WO 00/15188 A1 discloses the use of peptides of general sequence X-Thr-Thr-Lys-Y, wherein in particular X = lysine and Y = serine, in cosmetic or dermopharmaceutical compositions. These peptides can be used in mutual combination. In order to enhance their activity and their stability, the peptides are chemically modified to increase their lipophilicity, by grafting on the N-terminal amine of X, either a fatty acid chain, or by esterification or amidation of the C-terminal carboxyl group of Y. The peptides can be obtained by synthesis, biotechnology or controlled hydrolysis or plant proteins. The resulting compositions are advantageously used for stimulating healing, hydrating or all skin treatments. They are particularly active against formation or deterioration of wrinkles and against all the consequences of skin ageing, whether natural or induced (heliodermia, pollution), as well as for dry skin.

The EP 2 803 351 A1 discloses a continuous process for preparing microspheres and microspheres prepared thereby, and in particular, a process for preparing microspheres comprising steps of injecting a first emulsion and a second emulsion at the same time to form microspheres instantaneously, applying high pressure to the microspheres formed, and injecting the microspheres into an agitator, wherein the steps can be carried out continuously, and microspheres prepared thereby.

## [DETAILED DESCRIPTION OF THE INVENTION]

### [Technical Problem]

The present disclosure has been devised to resolve the above-mentioned in vivo stability problems of collagen peptide and improve the efficacy of the polycaprolactone microsphere filler, and it is an object of the present disclosure to provide a collagen peptide-containing polycaprolactone microsphere which, when applied to a living body, rapidly exhibits effects after treatment and maintains the effects for a long period of time, and a filler comprising the same and a method for preparing the same.

### [Technical Solution]

The problem of the present invention is solved by a collagen peptide-containing polycaprolactone microsphere according to the independent claim 1 as well as by a method for preparing a collagen peptide-containing polycaprolactone microsphere according to the independent claim 3 as well as by a polycaprolactone microsphere filler according to the independent claim 8. The dependent claims refer to further advantageous developments of the present invention.

### [ADVANTAGEOUS EFFECTS]

The filler comprising the collagen peptide-containing polycaprolactone microsphere according to the present disclosure not only exhibits a rapid collagen formation effect when applied to a living body and exhibits a high tissue restoration property, but also maintains the effects for a long period of time, thereby showing excellent restoration or volume expansion of soft tissues such as cheeks, breasts, nose, lips, and buttocks, or wrinkle improvement properties.

### [BRIEF DESCRIPTION OF THE DRAWINS]

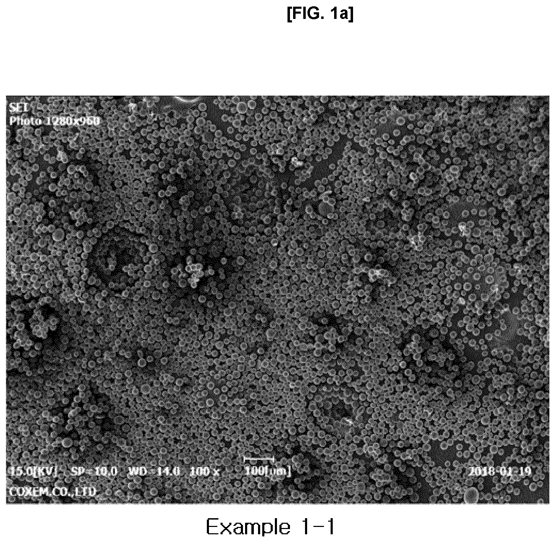

FIG. 1a is a photograph of the shape of the polycaprolactone microsphere containing palmitoyl-KTTKS (Palmitoyl-KTTKS) prepared according to Example 1-1, taken by an optical microscope. As can be seen from the photograph, it can be confirmed that the produced microspheres maintain a spherical shape and the particle size can be adjusted depending on the diameter of the membrane used in the production.

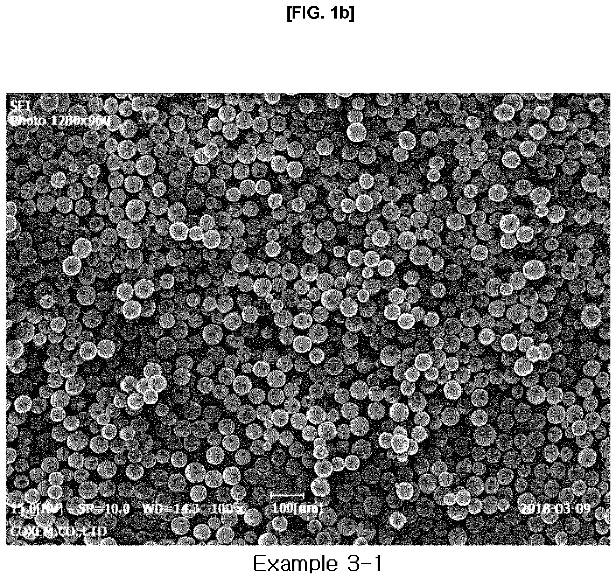

FIG. 1b is a photograph of the shape of the polycaprolactone microsphere containing palmitoyl-KTTKS prepared by using excess ascorbic acid according to Comparative Example 3-1, taken by an optical microscope. As can be seen from the photograph, it can be confirmed that the produced microspheres maintain a spherical shape and the particle size can be adjusted depending on the diameter of the membrane used in the production.

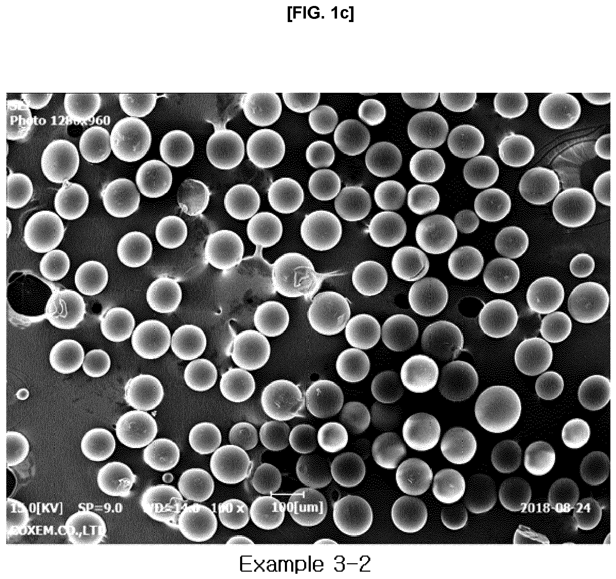

FIG. 1c is a photograph of the shape of the polycaprolactone microsphere containing palmitoyl-KTTKS prepared by using excess ascorbic acid according to Comparative Example 3-2, taken by an optical microscope. As can be seen from the photograph, it can be confirmed that the produced microspheres maintain a spherical shape and the particle size can be adjusted depending on the diameter of the membrane used in the production.

## [DETAILED DESCRIPTION OF THE EMBODIMENTS]

Hereinafter, the present disclosure will be described in more detail.

The collagen peptide according to the present disclosure refers to a peptide that exhibits a collagen regeneration promotion effect in vivo, and may be at least one selected from the group consisting of KTTKS, GHK, AHK, and derivatives thereof. Non-limiting examples of derivatives of the collagen peptides include palmitoyl-KTTKS, GHK-Cu, AHK-Cu, and the like. Preferably, KTTKS, palmitoyl-KTTKS, GHK, AHK and mixtures thereof can be used. More preferably, KTTKS or palmitoyl-KTTKS can be used.

The content (encapsulation amount) of collagen peptide in the polycaprolactone microsphere of the present disclosure is 0.01 to 7% by weight, preferably 0.05 to 6 % by weight, based on the total weight of the microsphere. This amount of encapsulation is optimized such that the characteristic physiological activity of collagen peptide can exhibit a synergistic effect at the injected site while ensuring the in vivo stability of collagen peptide. When the encapsulation amount of collagen peptide is less than 0.01 % by weight, the synergistic promotion effect of collagen production that can be expressed by collagen peptide does not appear sufficiently. When the encapsulation amount of the collagen peptide exceeds 7 % by weight, the encapsulation efficiency of collagen peptide in the polycaprolactone microsphere decreases, and the collagen peptide forms non-uniform channels inside the microspheres, and the collagen peptide is rapidly released through diffusion into the formed channel, which is not preferable.

The collagen peptide-containing polycaprolactone microsphere of the present disclosure is prepared using polycaprolactone in which an inherent viscosity of polycaprolactone, which is a biodegradable polymer, is 0.16 to 1.90 dL/g. The inherent viscosity of polycaprolactone used herein refers to the inherent viscosity measured in chloroform at 25 °C using a Ubbelohde viscometer. Examples of the above polycaprolactone polymer include Resormer C209, C212 and C217 available from Evonik, Purasorb PC02, PC04, PC08, PC12 and PC17 available from Corbion, and the like. When the inherent viscosity of the polycaprolactone used is lower than 0.16 dL/g, due to its low viscosity, polycaprolactone microsphere is not well formed, or the microsphere is decomposed too quickly when injected in a living body, and thus, the initial release of collagen peptide may be rapidly increased. When the viscosity exceeds 1.90 dL/g, the rate of degradation of the microsphere is decreased when injected in a living body, and due to the influence of the slow degradation rate of the polymer, the rate at which the collagen peptide in the microsphere spreads into the living body is reduced, making it difficult to exhibit a sufficient collagen production promoting effect.

The collagen peptide-containing polycaprolactone microsphere according to the present disclosure has an average particle size of 10 $\mu$m or more and 100 $\mu$m or less, for example, preferably 10 to 30 $\mu$m, 10 to 50 $\mu$m, or 10 to 100 $\mu$m, 20 to 50 $\mu$m, 30 to 60 $\mu$m, or 40 to 70 $\mu$m. The average particle size used herein refers to a median diameter as the particle size corresponding to 50 % of the volume% in the particle size distribution curve, and is represented by D50 or D (v, 0.5).

When the average particle size of the collagen peptide-containing polycaprolactone microsphere is less than 10 $\mu$m, it may be phagocytosed by macrophages when administered in the living body. When the average particle size is larger than 100 $\mu$m, the injectability is decreased when injected with a syringe, and the injection needle becomes thicker, causing more pain during injection, which is not preferable.

Preferably, the collagen peptide-containing polycaprolactone microsphere according to the present disclosure has a uniform particle distribution. The microsphere having a uniform particle distribution has less deviation in the residual amount of the syringe and needle during injection and less clogging phenomenon of the injection needle as compared with a non-uniform microsphere, and thus, a fine injection needle can be used. The size distribution degree or span value of the polycaprolactone microspheres of the present disclosure is 1.0 or less. More preferably, the size distribution is 0.8 or less. The size distribution or span value used herein is an index indicating the uniformity of the particle size of the microsphere, and means a value determined by the formula of the size distribution (span value) = (Dv0.9-Dv0.1) /Dv0.5. Here, Dv0.1 means a particle size corresponding to 10 % of the volume % in the particle size distribution curve of the microsphere, Dv0.5 means a particle size corresponding to 50 % of the volume% in the particle size distribution curve of the microsphere, and Dv0.9 means a particle size corresponding to 90 % of the volume% in the particle size distribution curve of the microsphere. The collagen peptide-containing polycaprolactone microsphere according to the present disclosure is characterized by exhibiting a uniform size distribution while exhibiting a particle size of 10 $\mu$m or more and 100 $\mu$m or less, thereby reducing needle clogging and improving injectability.

The particle size range and span values as described above are optimized so as to include the amount of encapsulation that allows collagen peptide in the polycaprolactone microsphere to be eluted in an appropriate amount for a long period of time. The polycaprolactone microparticles containing collagen peptide according to the present disclosure having such characteristics are characterized by releasing an effective amount of collagen peptide over a long period of time. Specifically, the collagen peptide-containing polycaprolactone microsphere according to the present disclosure gradually release collagen peptide for preferably 30 days, more preferably 35 days to 42 days, even more preferably 56 days to 60 days.

In addition, the collagen peptide according to the present disclosure shows a cumulative elution rate of 0.1 to 10% until 1 day after elution, when measuring the cumulative elution rate of microspheres in vitro. It shows a cumulative elution rate of 40 to 65% until 14 days, and a cumulative elution rate of 70 to 100% until 56 days.

The collagen peptide-containing polycaprolactone microsphere according to the present disclosure can be prepared, for example, using the "solvent extraction/evaporation method", without being limited thereto.

As a specific example of a method for preparing the collagen peptide-containing polycaprolactone microsphere according to the present disclosure, such preparation method comprises the steps of: (a) preparing a dispersed phase by dissolving polycaprolactone in a first solvent and dissolving collagen peptide in a second solvent to prepare each solution, and then uniformly mixing the two solutions to prepare a single solution; (b) preparing an emulsion by mixing the dispersed phase with an aqueous solution (continuous phase) containing a surfactant; (c) forming a microsphere by extracting and evaporating the organic solvent from the dispersed phase into a continuous phase in the emulsion prepared in step (b); and (d) recovering the microsphere from the continuous phase of step (c) to prepare a collagen peptide-containing polycaprolactone microsphere.

In step (a), the inherent viscosity of polycaprolactone is in the range of 0.16 to 1.90 dL/g.

The first solvent used for dissolving polycaprolactone in step (a) preferably has properties that are immiscible with water. By utilizing the properties of the organic solvent that are immiscible with water, the dispersed phase can be homogeneously mixed and dispersed in an aqueous solution containing a surfactant that is a continuous phase in step (b) described below, thereby forming an emulsion. The type of the solvent that dissolves polycaprolactone is not particularly limited, and preferably, it may be selected from the group consisting of dichloromethane, chloroform, ethyl acetate, methyl ethyl ketone, and a mixed solvent thereof. More preferably, dichloromethane, ethyl acetate or a mixed solvent thereof can be used.

The second solvent for dissolving collagen peptide in step (a) may be selected from the group consisting of methyl alcohol, ethyl alcohol, acetone, acetonitrile, dimethyl sulfoxide, dimethylformamide, N-methylpyrrolidone, acetic acid, and a mixture thereof. Preferably, methyl alcohol, dimethyl sulfoxide or a mixed solvent thereof can be used.

In step (a), a polycaprolactone and a collagen peptide solution are mixed to prepare a uniform mixed solution, thereby preparing a dispersed phase. Preferably, the mixed solution of polycaprolactone and collagen peptide is homogeneously dissolved so that collagen peptide is suitably encapsulated in the polycaprolactone microsphere. As an example, when using dichloromethane as a solvent for polycaprolactone and using dimethyl sulfoxide as a solvent for collagen peptide, the amount of methyl alcohol used is preferably 2 % by weight to 50 % by weight relative to the weight of dichloromethane. When the amount of methyl alcohol is less than 2 % by weight, it is highly likely that collagen peptide is precipitated due to the decrease of solubility by dichloromethane. When it exceeds 50 % by weight, it is highly likely that polycaprolactone is precipitated by methyl alcohol, which is not preferable.

In step (b), the method for homogeneously mixing the dispersion phase and the aqueous solution containing the surfactant is not particularly limited, and preferably, the mixing can be performed using a high-speed stirrer, an in-line mixer, a membrane emulsification method, a microfluidic emulsification method, or the like. As an example, when performing the mixing using a membrane emulsification method, the dispersed phase prepared in step (a) is passed through a membrane having uniformly sized micropores and transferred to a continuous phase containing a surfactant to prepare an emulsion.

The type of the surfactant used of step (b) is not particularly limited, and the surfactant can be used without limitation as long as it can help the dispersion phase to form a stable droplet emulsion within the continuous phase. Preferably, the surfactant may be selected from the group consisting of methyl cellulose, polyvinylpyrrolidone, carboxymethylcellulose, lecithin, gelatin, polyvinyl alcohol, polyoxyethylene sorbitan fatty acid ester, polyoxyethylene castor oil derivatives, and mixtures thereof. Most preferably, polyvinyl alcohol can be used.

In step (b), the content of the surfactant in the continuous phase containing the surfactant may be 0.01 w/v% to 20 w/v%, preferably 0.1 w/v% to 5 w/v% based on the total volume of the continuous phase containing the surfactant. When the content of surfactant is less than 0.01 w/v%, a dispersed phase or emulsion in the form of droplets may not be formed in the continuous phase. When the content of surfactant exceeds 20 w/v%, it may be difficult to remove the surfactant after fine particles are formed in the continuous phase due to excessive surfactant. In an embodiment of the present disclosure, the collagen peptide-containing polycaprolactone microsphere is prepared using 1 to 5 w/v% of polyvinyl alcohol.

In step (c), the emulsion comprising a dispersed phase in the form of droplets and a continuous phase containing a surfactant can be stirred for 48 hours or less, preferably 1 to 36 hours, more preferably for about 3 to 24 hours, while maintaining a temperature below the boiling point of the organic solvent, for example, not limited thereto, 5 to 39.6 °C, preferably 10 to 35 °C, more preferably 15 to 30 °C, thereby removing the organic solvent. The stirring speed is not particularly limited, but 10 to 300 rpm is suitable. A part of the organic solvent extracted into the continuous phase can be evaporated from the surface of the continuous phase. As the organic solvent is removed from the solution in the form of droplets, the dispersed phase in the form of droplets is solidified to form microspheres, and thereby, the form of a suspension containing microspheres (microsphere suspension) is obtained.

In step (c), in order to more efficiently remove the organic solvent, the temperature of the continuous phase may be heated for a certain period of time.

In step (d), the method for recovering polycaprolactone microspheres can be performed using several known techniques, and for example, a method such as filtration or centrifugation can be used.

Between steps (c) and (d), the residual surfactant may be removed through filtration and washing, and then filtration may be performed again to recover the microspheres.

The washing step for removing the residual surfactant can be usually performed using water, and the washing step can be repeated several times.

Further, as described above, when the emulsion is formed using the high-speed stirrer and the in-line mixer in step (b), a uniform microsphere can be obtained by additionally using a sieving process between steps (c) and (d). The sieving process can be performed using a known technique, and the microspheres of small particles and large particles can be filtered using a sieve membrane having different sizes to obtain microspheres of uniform size.

According to another aspect of the present disclosure, there is provided a filler comprising the collagen peptide-containing polycaprolactone microsphere of the present disclosure; and a pharmaceutically acceptable aqueous carrier.

As the pharmaceutically acceptable aqueous carrier, for example, an aqueous solution for injection such as purified water, physiological saline, or phosphate buffer may be used. Further, the filler may, in addition to the collagen peptide-containing polycaprolactone microsphere and the pharmaceutically acceptable aqueous carrier, include at least one selected from the group consisting of cellulose derivatives such as carboxymethylcellulose (CMC) and hydroxypropylmethylcellulose (HPMC), solutes such as hyaluronic acid, lidocaine, polydeoxyribonucleotide (PDRN), polynucleotide (PN), and lubricants such as glycerin, if necessary, but is not limited thereto. Preferred solutes are carboxymethylcellulose or hyaluronic acid.

In one embodiment, the content of each component included in the filler comprising the collagen peptide-containing polycaprolactone microsphere of the present disclosure may be 2 to 50 % by weight of the collagen peptide-containing polycaprolactone microsphere (the content of collagen peptide in the polycaprolactone microparticle is 0.01 to 7 % by weight), 15 to 97.9 % by weight of the pharmaceutically acceptable aqueous carrier, 0.1 to 5 % by weight of the solute and 0 to 48 % by weight of the lubricant, based on the total 100 % by weight of the filler formulation, but is not limited thereto. When hyaluronic acid is added as a solute, hyaluronic acid having a crosslinking rate of 0 to 5% may be used.

The filler comprising the collagen peptide-containing polycaprolactone microsphere according to the present disclosure not only exhibits a rapid collagen-forming effect at the treated site immediately after the treatment, and exhibits a tissue repair property showing a natural and ideal volume feeling, but also maintains the in vivo stability of collagen peptide, has good injectability, and exhibits excellent effects for a long period of time, and therefore, can be very usefully used for cosmetic or therapeutic purposes.

As a specific example, the filler including these polycaprolactone microspheres can be used for filling of biological tissues, winkle improvement through filling of wrinkles, remodeling of the face, or restoring or increasing the volume of soft tissues such as lips, nose, buttocks, cheeks or chest. The filler including the polycaprolactone microspheres may be administered in a dosage form suitable for this use, and preferably may be an injection.

In another aspect, the present disclosure provides a prefilled syringe filled with a filler comprising the polycaprolactone microspheres.

Hereinafter, the present disclosure will be described in more detail by way of examples. However, these examples are for illustrative purposes only, and the scope of the present disclosure is not limited thereto.

## Example 1: Preparation of microsphere with encapsulated collagen peptide

The disperse phase was prepared by completely dissolving 9.99 g of biocompatible polymer Purasorb PC 04 (manufacturer: Corbion, The Netherlands) and 0.01 g of palmitoyl-KTTKS (manufacturer: Incospharm, Korea) in 39.96 g of dichloromethane (manufacturer: J.T. Baker, USA) and 2.02 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 3200 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 1-1: Preparation of microsphere with encapsulated collagen peptide

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 4000 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

**Example 1-2: Preparation of microsphere with encapsulated collagen peptide**

The disperse phase was prepared by completely dissolving 9.9 g of biocompatible polymer Purasorb PC 04 (manufacturer: Corbion, The Netherlands) and 0.1 g of palmitoyl-KTTKS (manufacturer: Incospharm, Korea) in 39.6 g of dichloromethane (manufacturer: J.T. Baker, USA) and 4.0 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 5000 mL of the continuous phase was supplied to an emulsification apparatus equipped with 10 µm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

**Example 1-3: Preparation of microsphere with encapsulated collagen peptide**

The disperse phase was prepared by completely dissolving 9.5 g of biocompatible polymer Purasorb PC 04 (manufacturer: Corbion, The Netherlands) and 0.5 g of palmitoyl-KTTKS (manufacturer: Incospharm, Korea) in 38.0 g of dichloromethane (manufacturer: J.T. Baker, USA) and 6.19 mL of methyl alcohol (manufacturer: Sigma Aldrich, USA), respectively, and mixing the two solutions. As the continuous phase, a 3 w/v% polyvinyl alcohol aqueous solution (viscosity: 4.8 to 5.8 mPa·s) was used, and 5400 mL of the continuous phase was supplied to an emulsification apparatus equipped with 10 µm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25°C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

**Example 1-4: Preparation of microsphere with encapsulated collagen peptide**

The disperse phase was prepared by completely dissolving 9 g of biocompatible polymer Purasorb PC 04 (manufacturer: Corbion, The Netherlands) and 1 g of palmitoyl-KTTKS (manufacturer: Incospharm, Korea) in 36.0 g of dichloromethane (manufacturer: J.T. Baker, USA) and 10.77 mL of methyl alcohol (manufacturer: Sigma Aldrich, USA), respectively, and mixing the two solutions. As the continuous phase, a 3 w/v% polyvinyl alcohol aqueous solution (viscosity: 4.8 to 5.8 mPa·s) was used, and 4800 mL of the continuous phase was supplied to an emulsification apparatus equipped with 10 µm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25°C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

### Example 2: Preparation of microsphere with encapsulated collagen peptide

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 24.95 g of dichloromethane (manufacturer: J.T. Baker, USA) and 1.57 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 2500 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

### Example 2-1: Preparation of microsphere with encapsulated collagen peptide

The disperse phase was prepared by completely dissolving 9.98 g of biocompatible polymer Purasorb PC 12 (manufacturer: Corbion, The Netherlands) and 0.02 g of palmitoyl-KTTKS (manufacturer: Incospharm, Korea) in 55.44 g of dichloromethane (manufacturer: J.T. Baker, USA) and 3.5 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 6700 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

### Example 2-2: Preparation of microsphere with encapsulated collagen peptide

The disperse phase was prepared by completely dissolving 9.98 g of biocompatible polymer Purasorb PC 17 (manufacturer: Corbion, The Netherlands) and 0.02 g of palmitoyl-KTTKS (manufacturer: Incospharm, Korea) in 83.16 g of dichloromethane (manufacturer: J.T. Baker, USA) and 5.25 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 12500 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

### Example 3: Preparation of microsphere with encapsulated collagen peptide

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 4000 mL of the continuous phase was supplied to an emulsification apparatus equipped with a porous membrane having a diameter of 5 μm, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25°C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 3-1: Preparation of microsphere with encapsulated collagen peptide

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 4000 mL of the continuous phase was supplied to an emulsification apparatus equipped with a porous membrane having a diameter of 30 $\mu$m, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25°C.

When the injection of the dispersed phase was completed, the microsphere suspension was stirred at 25°C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 3-2: Preparation of microsphere with encapsulated collagen peptide

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 2 w/v% polyvinyl alcohol aqueous solution (viscosity: 4.8 to 5.8 mPa·s) was used, and 4000 mL of the continuous phase was supplied to an emulsification apparatus equipped with a porous membrane having a diameter of 40 $\mu$m, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25°C.

When the injection of the dispersed phase was completed, the microsphere suspension was stirred at 25°C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 4: Preparation of microsphere with encapsulated collagen peptide using high-speed stirrer

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 1 w/v% polyvinyl alcohol aqueous solution (viscosity: 4.8 to 5.8 mPa·s) was used, and 4000 mL of the continuous phase was placed in a preparation container, and while stirring the equipped high-speed mixer at a speed of 4500 rpm, the dispersed phase was injected at a flow rate of 7 mL per minute. The microsphere suspension was stirred at a speed of 150 rpm. The temperature of the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, the microspheres were screened using a mesh with 25 $\mu$m and 150 $\mu$m sieves at the same time, and then lyophilized.

## Example 5: Preparation of polycaprolactone microsphere with encapsulated GHK-Cu

The disperse phase was prepared by completely dissolving 9.98 g of biocompatible polymer Purasorb PC 04 (manufacturer: Corbion, The Netherlands) and 0.02 g of GHK-Cu (manufacturer: Incospharm, Korea) as a bioactive material in 39.92 g of dichloromethane (manufacturer: J.T. Baker, USA) and 60 μL of phosphate buffer (pH 7.2), respectively, and vortexing the two solutions. As the continuous phase, a 1 w/v% polyvinyl alcohol aqueous solution (viscosity: 4.8 to 5.8 mPa·s) was used, and 4800 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsfication apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 5-1: Preparation of polycaprolactone microsphere with encapsulated AHK-Cu

The disperse phase was prepared by completely dissolving 9.98 g of biocompatible polymer Purasorb PC 04 (manufacturer: Corbion, The Netherlands) and 0.02 g of AHK-Cu (manufacturer: Incospharm, Korea) as a bioactive material in 39.92 g of dichloromethane (manufacturer: J.T. Baker, USA) and 70 μL of phosphate buffer (pH 7.2), respectively, and vortexing the two solutions. As the continuous phase, a 1 w/v% polyvinyl alcohol aqueous solution (viscosity: 4.8 to 5.8 mPa·s) was used, and 4800 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsfication apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

## Example 6: Preparation of polycaprolactone microsphere filler using carboxymethylcellulose as a solute

The polycaprolactone microsphere filler was prepared by preparing a solution for the suspension of microspheres and then mixing the microspheres. Specifically, 2 g of carboxymethylcellulose (manufacturer: Ashland, USA) was added to a phosphate buffer at 75 °C, dissolved and cooled with stirring at 100 rpm for 3 hours. When the temperature of the solution reached 25 °C, 18 g of glycerin was added thereto and finally, the polycaprolactone microsphere with encapsulated collagen peptide according to Examples 1-2 were mixed at 30 % (w/w) to complete the preparation of a polycaprolactone microsphere filler.

## Example 6-1: Preparation of polycaprolactone microsphere filler using hyaluronic acid as a solute

The polycaprolactone microsphere filler was prepared by preparing a solution for the suspension of microspheres and then mixing the microspheres. 1 g of hyaluronic acid (manufacturer: Bloomage Freda Biopharm, China) was added to a phosphate buffer at 55 °C, dissolved and cooled. When the temperature of the solution reached 25 °C, 18 g of glycerin was added thereto and finally, the polycaprolactone microsphere with encapsulated collagen peptide according to Examples 1-2 were mixed at 30 % (w/w) relative to the total weight of the polycaprolactone microsphere to complete the preparation of a polycaprolactone microsphere filler.

## Comparative Example 1: Preparation of microsphere with encapsulated collagen peptide

The disperse phase was prepared by completely dissolving 8 g of biocompatible polymer Purasorb PC 04 (manufacturer: Corbion, The Netherlands) and 2 g of palmitoyl-KTTKS (manufacturer: Incospharm, Korea) in 32.0 g of dichloromethane (manufacturer: J.T. Baker, USA) and 15.32 mL of methyl alcohol (manufacturer: Sigma Aldrich, USA), respectively, and mixing the two solutions. As the continuous phase, a 3 w/v% polyvinyl alcohol aqueous solution (viscosity: 4.8 to 5.8 mPa·s) was used, and 4800 mL of the continuous phase was supplied to an emulsification apparatus equipped with 10 μm diameter porous membrane, and at the same time, the prepared dispersed phase was injected to prepare a microsphere. The prepared microsphere suspension was placed in a preparation container and stirred at a speed of 150 rpm. The temperature of the membrane emulsification apparatus and the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

**Comparative Example 2: Preparation of microsphere with encapsulated bioactive peptide using high-speed stirrer**

The disperse phase was prepared by completely dissolving 9.98 g of biocompatible polymer Purasorb PC 04 (manufacturer: Corbion, The Netherlands) and 0.02 g of palmitoyl-KTTKS (manufacturer: Incospharm, Korea) in 39.92 g of dichloromethane (manufacturer: J.T. Baker, USA) and 2.52 mL of methyl alcohol (manufacturer: Sigma Aldrich, USA), respectively, and mixing the two solutions. As the continuous phase, a 1 w/v% polyvinyl alcohol aqueous solution (viscosity: 4.8 to 5.8 mPa·s) was used, and 4000 mL of the continuous phase was placed in a preparation container, and while stirring the equipped high-speed mixer at a speed of 4500 rpm, the dispersed phase was injected at a flow rate of 7 mL per minute. The microsphere suspension was stirred at a speed of 150 rpm. The temperature of the preparation container was maintained at 25 °C.

When the injection of the dispersed phase was completed, the microsphere suspension was stirred at 25 °C for 12 hours at a speed of 150 rpm to remove the organic solvent. When the removal of the organic solvent was completed, the microsphere suspension was repeatedly washed with deionized water several times to remove and obtain residual polyvinyl alcohol, and the obtained microspheres were lyophilized.

**Experimental Example 1: Morphological analysis through electron microscope**

In this experiment, the morphological characteristics of the prepared microspheres were analyzed through an electron microscope. The experimental procedure is as follows. 5 mg of the microspheres prepared in Examples 1-1, 3-1, and 3-2 were placed on an aluminum stub attached with carbon tape, and platinum coating was performed using ION-COATER (COXEM, Korea). An aluminum stub was mounted on a scanning electron microscope (COXEM EM-30, Korea), and the morphological characteristics of the microspheres were observed with an acceleration voltage of 15 kV, and the results are shown in FIG. 1a (Example 1-1) and FIG. 1b (Example 3-1) and FIG. 1c (Example 3-2).

As shown in FIG. 1a, FIG. 1b and FIG. 1c, it was confirmed that the microspheres maintained a spherical shape and the particle size could be adjusted depending on the membrane diameter used in the production.

**Experimental Example 2: Particle size analysis of microsphere using a laser diffraction method**

This experiment was carried out to quantitatively measure the average particle size and distribution of microspheres and confirm the uniformity of particles. The experimental procedure is as follows.

50 mg of microspheres were mixed with 1 mL of deionized water, mixed with a vortex mixer for 20 seconds, then placed in an ultrasonic generator for 1 minute and dispersed. The microsphere dispersion was placed in a particle size analyzer (Microtrac Bluewave, Japan) and measured for 20 seconds. As an index of particle size uniformity, the span value was determined by the following Formula.

【Formula 1】

$$\text{Span Value} = (D_{v,0.9} - D_{v,0.1}) / D_{v,0.5}$$

[Table 1]

|  | $D_{v,0.5}$ (μm) | Span Value |
|---|---|---|
| Example 1 | 38.9 | 0.67 |
| Example 1-1 | 37.2 | 0.62 |
| Example 1-2 | 36.3 | 0.74 |
| Example 1-3 | 36.7 | 0.68 |
| Example 1-4 | 35.1 | 0.70 |
| Example 2 | 34.7 | 0.62 |
| Example 2-1 | 36.9 | 0.65 |

(continued)

|  | $D_{v,0.5}$ (µm) | Span Value |
|---|---|---|
| Example 2-2 | 36.8 | 0.66 |
| Example 3 | 15.2 | 0.68 |
| Example 3-1 | 56.8 | 0.72 |
| Example 3-2 | 103.3 | 0.71 |
| Example 4 | 33.5 | 0.92 |
| Comparative Example 1 | 32.4 | 0.69 |
| Comparative Example 2 | 37.7 | 1.38 |

As shown in Table 1 above, it was confirmed that Example 1, Example 1-1, Example 1-2, Example 1-3, Example 1-4 and Comparative Example 1 had similar average particle sizes through $D_{v,0.5}$ and span value measured by a particle size analyzer. Example 1-1, Example 3, Example 3-1 and Example 3-2 were microspheres prepared using membranes having different diameters, respectively, and had an average particle size of about 10 to 100 µm. At this time, all microspheres had a span value of 1.0 or less and thus a uniform particle distribution. From this, it was confirmed that it was possible to prepare microspheres having an average particle size of 10 to 100 µm while having a span value of 1.0 or less and thus a uniform particle distribution.

In the case of Example 2, Example 2-1, and Example 2-1, the types of the polymers used for preparing the dispersed phase were different, but it was confirmed that it was possible to adjust the size of the prepared dispersed phase and the membrane size of the apparatus for producing microspheres, thereby adjusting it to similar average particle sizes.

In the case of Example 1-1, Example 4, and Comparative Example 2, there was a difference in the method of mixing the dispersed phase with an aqueous solution containing a surfactant, and the prepared microspheres showed different average particle sizes and span values depending on the method. In particular, in the case of Example 4 and Comparative Example 2 using a high-speed stirrer, the span value could be adjusted to 1.0 or less through sieving, which is a microparticle screening process. In the case of Comparative Example 2 without a separate sieving process, it had a span value of 1.38 and thus a relatively wide particle size distribution.

**Experimental Example 3: Analysis of collagen peptide content**

This experiment was carried out to quantitatively analyze the collagen peptide encapsulated in the microsphere, and quantitative analysis was performed using liquid chromatography mass spectrometry (LC-MS/MS). The experimental procedure is as follows.

100 mg of the microspheres was completely dissolved in a mixed solution of dimethyl sulfoxide and methyl alcohol, and then diluted with a mobile phase. The mobile phase used for the analysis was used by mixing ammonium acetate solution and acetonitrile in a ratio of 30:70 (v/v), and was prepared to contain 0.05 % acetic acid. A C8 column (2.0 x 100 mm, 5 µm) was used for this measurement. Th amount of encapsulation measured is shown in Table 2 below.

[Table 2]

|  | Encapsulation amount (wt%) |
|---|---|
| Example 1 | 0.09 |
| Example 1-1 | 0.17 |
| Example 1-2 | 0.83 |
| Example 1-3 | 3.99 |
| Example 1-4 | 5.54 |
| Example 2 | 0.17 |
| Example 2-1 | 0.19 |
| Example 2-2 | 0.18 |
| Example 3 | 0.17 |
| Example 3-1 | 0.18 |

(continued)

|  | Encapsulation amount (wt%) |
|---|---|
| Example 3-2 | 0.18 |
| Example 4 | 0.16 |
| Comparative Example 1 | 8.92 |
| Comparative Example 2 | 0.18 |

As shown in Table 2 above, the encapsulation amount (content) increased in proportion to the amount of collagen peptide used for preparing the dispersed phase, and the encapsulation efficiency decreased in inverse proportion to the amount of collagen peptide used. The contents of collagen peptide of Example 1-1, Example 3, Example 3-1, and Example 3-2 were similar, confirming that the particle size did not affect the encapsulation rate.

**Experimental Example 4: In vitro release behavior of collagen peptide**

This experiment was conducted to confirm the drug release performance of collagen peptide encapsulated in the polycaprolactone microsphere, and the experimental procedure is as follows.

100 mg of microspheres produced in Examples 1-1, 1-2, 1-4, 2 and 2-2, and Comparative Example 1 were placed in a 250 mL wide mouth bottle containing 200 mL of a 10 mM HEPES buffer. At a predetermined time interval, 1 mL of the solution was taken from a wide mouth bottle and an equal amount of fresh HEPES buffer was added thereto. The collected solution was placed in a 1.5 mL tube, centrifuged at 13000 rpm for 5 minutes, and then the elution rate was confirmed by liquid chromatography mass spectrometry similarly to the analysis method of the collagen peptide content.

[Table 3]

| | Cumulative elution rate of collagen peptide in vitro (%) | | | | | | | | | | |
|---|---|---|---|---|---|---|---|---|---|---|---|
| | 1 day | 4 day | 7 day | 11 day | 14 day | 21 day | 28 day | 35 day | 42 day | 49 day | 56 day |
| Example 1-1 | 0.7 | 3.7 | 16.1 | 27.4 | 44.1 | 59.2 | 71.1 | 83.7 | 94.5 | 95.1 | 95.5 |
| Example 1-2 | 1.1 | 5.3 | 19.4 | 31.8 | 49.2 | 65.5 | 79.3 | 90.5 | 96.2 | 97.6 | 97.8 |
| Example 1-4 | 5.4 | 11.2 | 20.0 | 37.9 | 62.6 | 80.3 | 91.5 | 97.5 | | | |
| Example 2 | 2.9 | 10.6 | 21.9 | 40.0 | 58.4 | 74.2 | 87.1 | 94.3 | 97.2 | | |
| Example 2-2 | 0 | 0.7 | 1.1 | 1.6 | 4.8 | 9.2 | 19.0 | 32.8 | 47.7 | 64.5 | 78.6 |
| Comparative Example 1 | 10.8 | 68.9 | 84.6 | 95.7 | 96.1 | | | | | | |

As shown in Table 3 above, through the cumulative elution rate of Example 1, Example 1-2 and Example 1-4 according to the present disclosure, it was confirmed that the release of the collagen peptide encapsulated in the polycaprolactone microspheres gradually released over 56 days.

In more detail, it was confirmed that the larger the amount of collagen peptide encapsulated in the microsphere, the higher the initial release and the faster the release rate. On the other hand, in the case of the microsphere produced according to Comparative Example 1 and not according to the present invention, it exhibited a high elution rate from the initial stage of elution, and showed a behavior of rapidly releasing about 69% of the encapsulated collagen peptide for 4 days. In about 14 days, it was confirmed that the elution of the collagen peptide was almost completed and the duration was not long. Considering that the amount of collagen peptide and solvent used for preparing the dispersed phase was excessively higher than in Example 1, Example 1-2 and Example 1-4 and the encapsulation efficiency was also decreased, it was predicted that collagen peptides formed non-uniform channels inside the microspheres, and the collagen peptide was rapidly released through diffusion into the formed channel.

Through the cumulative elution rate results of Examples 1-1, 2 and 2, it was confirmed that when the viscosity of the polymer used for preparing the dispersed phase increased, the release rate of the encapsulated collagen peptide decreased. This was predicted to be because not only the degradation rate of the microsphere decreases with the increase of the molecular weight of the polymer, but also the diffusion rate of the collagen peptide in the microsphere into the eluate decreases due to the effect of the slow degradation rate of the polymer.

**Claims**

1. A collagen peptide-containing polycaprolactone microsphere comprising 0.01 to 7 % by weight of collagen peptide which exhibits a collagen regeneration promotion effect in vivo based on total 100 % by weight of the polycaprolactone microsphere containing collagen peptide and having an average particle size of 10 to 100 μm, wherein the polycaprolactone has an inherent viscosity of 0.16 to 1.90 dL/g;

   wherein the microsphere has a span value of 1.0 or less, and wherein the span value is calculated by following formula:

$$(\text{span value}) = (Dv0.9 - Dv0.1)/Dv0.5,$$

   wherein in said formula Dv0.1 means a particle size corresponding to 10 % of the volume% in the particle size distribution curve of the microsphere, Dv0.5 means a particle size corresponding to 50 % of the volume% in the particle size distribution curve of the microsphere, and Dv0.9 means a particle size corresponding to 90 % of the volume% in the particle size distribution curve of the microsphere.

2. The collagen peptide-containing polycaprolactone microsphere according to claim 1, wherein the collagen peptide is at least one selected from the group consisting of KTTKS, GHK, AHK, and derivatives thereof.

3. A method for preparing a collagen peptide-containing polycaprolactone microsphere, comprising the steps of:

   (a) preparing a single solution by dissolving polycaprolactone in a first solvent and dissolving collagen peptide which exhibits a collagen regeneration promotion effect in vivo in a second solvent to prepare each solution, and then uniformly mixing the two solutions to prepare a single solution;
   (b) preparing an emulsion by mixing the single solution obtained in step a) with an aqueous solution (continuous phase) containing a surfactant;
   (c) extracting organic solvents from the single solution as a dispersed phase into a continuous phase in the emulsion prepared in step (b) and evaporating said organic solvent from the surface of the continuous phase to form a microsphere; and
   (d) recovering the microsphere from the continuous phase of step (c) to prepare a polycaprolactone microsphere,

   when the emulsion is formed using high-speed stirrer in step (b), the method comprises an additional sieving process between steps (c) and (d) in order to obtain a uniform microsphere;
   wherein the polycaprolactone has an inherent viscosity of 0.16 to 1.90 dL/g;
   wherein the microsphere comprises 0.01 to 7 % by weight of collagen peptide based on total 100 % by weight of the polycaprolactone microsphere containing collagen peptide and has an average particle size of 10 to 100 μm;
   wherein the microsphere has a span value of 1.0 or less, and wherein the span value is calculated by following formula:

$$(\text{span value}) = (Dv0.9 - Dv0.1)/Dv0.5,$$

   wherein in said formula Dv0.1 means a particle size corresponding to 10 % of the volume% in the particle size distribution curve of the microsphere, Dv0.5 means a particle size corresponding to 50 % of the volume% in the particle size distribution curve of the microsphere, and Dv0.9 means a particle size corresponding to 90 % of the volume% in the particle size distribution curve of the microsphere.

4. The method for preparing a collagen peptide-containing polycaprolactone microsphere according to claim 3, wherein the collagen peptide is at least one selected from the group consisting of KTTKS, GHK, AHK, and derivatives thereof.

5. The method for preparing a collagen peptide-containing polycaprolactone microsphere according to claim 3,

   wherein the first solvent of step (a) is at least one selected from the group consisting of dichloromethane, chloroform, ethyl acetate, methyl ethyl ketone, and a mixed solvent thereof and
   wherein the second solvent of step (a) is at least one selected from the group consisting of methyl alcohol, ethyl alcohol, acetone, acetonitrile, dimethyl sulfoxide, dimethylformamide, N-methylpyrrolidone, acetic acid, and a mixture thereof.

6. The method for preparing a collagen peptide-containing polycaprolactone microsphere according to claim 3, wherein the surfactant of step (b) is at least one selected from the group consisting of methyl cellulose, polyvinylpyrrolidone, carboxymethylcellulose, lecithin, gelatin, polyvinyl alcohol, polyoxyethylene sorbitan fatty acid ester, polyoxyethylene castor oil derivatives, and mixtures thereof.

7. The method for preparing a collagen peptide-containing polycaprolactone microsphere according to claim 3 or 6, wherein the surfactant of step (b) is contained in an amount of 0.01 w/v% to 20 w/v% based on the total volume of the aqueous solution containing the surfactant.

8. A polycaprolactone microsphere filler comprising 2 to 50 % by weight of a collagen peptide-containing polycaprolactone microsphere which comprises 0.01 to 7 % by weight of the collagen peptide which exhibits a collagen regeneration promotion effect in vivo and has an average particle size of 10 to 100 $\mu$m; 0.1 to 5 % by weight of a solute; 0 to 48 % by weight of a lubricant; and 15 to 97.9 % by weight of a pharmaceutically acceptable aqueous carrier, based on total 100 % by weight of the polycaprolactone microsphere filler,

wherein the polycaprolactone has an inherent viscosity of 0.16 to 1.90 dL/g;
wherein the microsphere has a span value of 1.0 or less, and wherein the span value is calculated by following formula:

$$(\text{span value}) = (Dv0.9-Dv0.1)/Dv0.5,$$

wherein in said formula, Dv0.1 means a particle size corresponding to 10 % of the volume% in the particle size distribution curve of the microsphere, Dv0.5 means a particle size corresponding to 50 % of the volume% in the particle size distribution curve of the microsphere, and Dv0.9 means a particle size corresponding to 90 % of the volume% in the particle size distribution curve of the microsphere.

9. The polycaprolactone microsphere filler according to claim 8, wherein the collagen peptide is at least one selected from the group consisting of KTTKS, GHK, AHK, and derivatives thereof.

10. The polycaprolactone microsphere filler according to claim 8, wherein the solute is at least one selected from the group consisting of carboxymethylcellulose (CMC), hydroxypropylmethylcellulose (HPMC), hyaluronic acid, lidocaine, polydeoxyribonucleotide (PDRN), polynucleotide (PN), and a mixture thereof.

11. The polycaprolactone microsphere filler according to claim 8, wherein the lubricant is glycerin.

12. The polycaprolactone microsphere filler according to claim 8, wherein the pharmaceutically acceptable aqueous carrier is distilled water, physiological saline, or phosphate buffer.

13. The polycaprolactone microsphere filler according to claim 8,
wherein the filler is for improving wrinkles, repairing soft tissues or expanding volume, or correcting contours.

14. The polycaprolactone microsphere filler according to claim 8 or 13,
wherein the filler is for an injection.

15. A prefilled syringe filled with the polycaprolactone microsphere filler of any one of claims 8 to 14.

**Patentansprüche**

1. Kollagenpeptidhaltige Polycaprolacton-Mikrokügelchen, das bezogen auf insgesamt 100 Gewichtsprozent der Kollagenpeptid enthaltenden Polycaprolacton-Mikrokügelchen 0,01 bis 7 Gewichtsprozent Kollagenpeptid enthält, das in vivo eine kollagenregenerierende Wirkung zeigt, und das eine durchschnittliche Partikelgröße von 10 bis 100 $\mu$m aufweist, wobei das Polycaprolacton eine inhärente Viskosität von 0,16 bis 1,90 dL/g aufweist,

wobei das Mikrokügelchen einen Spannwert von 1,0 oder weniger aufweist und wobei der Spannwert nach der folgenden Formel berechnet wird:

$$(\text{Span-Wert}) = (Dv0,9 - Dv0,1)/Dv0,5,$$

wobei in dieser Formel das Dv0,1 eine Partikelgröße bedeutet, die 10 % des Volumenanteils in der Partikelgrößenverteilungskurve des Mikrokügelchen entspricht, das Dv0,5 eine Partikelgröße bedeutet, die 50 % des Volumenanteils in der Partikelgrößenverteilungskurve des Mikrokügelchen entspricht, und das Dv0,9 eine Partikelgröße bedeutet, die 90 % des Volumenanteils in der Partikelgrößenverteilungskurve des Mikrokügelchen entspricht.

2. Kollagenpeptidhaltige Polycaprolacton-Mikrokügelchen nach Anspruch 1, wobei das Kollagenpeptid mindestens eines ist, das aus der Gruppe ausgewählt worden ist, die ein KTTKS, ein GHK, ein AHK und deren Derivate umfasst.

3. Verfahren zur Herstellung eines kollagenpeptidhaltigen Polycaprolacton-Mikrokügelchen, welches die Schritte umfasst:

(a) ein Herstellen einer Einzellösung durch ein Auflösen von Polycaprolacton in einem ersten Lösungsmittel und ein Auflösen von einem Kollagenpeptid, das in vivo eine kollagenregenerationsfördernde Wirkung zeigt, in einem zweiten Lösungsmittel, um jede Lösung herzustellen, und ein anschließendes gleichmäßiges Mischen beider Lösungen, um eine Einzellösung herzustellen,
(b) ein Herstellen einer Emulsion durch ein Mischen der in dem Schritt a) erhaltenen Einzellösung mit einer wässrigen Lösung (kontinuierliche Phase), die ein Tensid enthält,
(c) ein Extrahieren organischer Lösungsmittel aus der Einzellösung als eine dispergierte Phase in eine kontinuierliche Phase in der in dem Schritt (b) hergestellten Emulsion und ein Verdampfen des organischen Lösungsmittels von der Oberfläche der kontinuierlichen Phase, um ein Mikrokügelchen zu bilden, und
(d) ein Gewinnen der Mikrokügelchen aus der kontinuierlichen Phase aus dem Schritt (c), um ein Polycaprolacton-Mikrokügelchen herzustellen,

wobei wenn die Emulsion mittels eines Hochgeschwindigkeitsrührers in dem Schritt (b) gebildet wird, das Verfahren einen zusätzlichen Siebprozess zwischen den Schritten (c) und (d) umfasst, um ein gleichmäßiges Mikrokügelchen zu erhalten,
wobei das Polycaprolacton eine inhärente Viskosität von 0,16 bis 1,90 dL/g aufweist,
wobei das Mikrokügelchen bezogen auf 100 Gewichtsprozent des kollagenpeptidhaltigen Polycaprolacton-Mikrokügelchen 0,01 bis 7 Gewichtsprozent Kollagenpeptid aufweist und eine durchschnittliche Teilchengröße von 10 bis 100 μm aufweist,
wobei das Mikrokügelchen einen Spannwert von 1,0 oder weniger aufweist und wobei der Spannwert nach der folgenden Formel berechnet wird:

$$(\text{Spannwert}) = (Dv0,9 - Dv0,1)/Dv0,5,$$

wobei in dieser Formel das Dv0,1 eine Partikelgröße bedeutet, die 10 % des Volumenprozentsatzes in der Partikelgrößenverteilungskurve des Mikrokügelchen entspricht, das Dv0,5 eine Partikelgröße bedeutet, die 50 % des Volumenanteils in der Partikelgrößenverteilungskurve des Mikrokügelchen entspricht, und das Dv0,9 eine Partikelgröße bedeutet, die 90 % des Volumenanteils in der Partikelgrößenverteilungskurve des Mikrokügelchen entspricht.

4. Verfahren zur Herstellung eines kollagenpeptidhaltigen Polycaprolacton-Mikrokügelchen nach Anspruch 3, wobei das Kollagenpeptid mindestens eines ist, das aus der Gruppe ausgewählt worden ist, die ein KTTKS, ein GHK, ein AHK und deren Derivate umfasst.

5. Verfahren zur Herstellung eines kollagenpeptidhaltigen Polycaprolacton-Mikrokügelchen nach Anspruch 3,

wobei das erste Lösungsmittel aus dem Schritt (a) mindestens eines ist, das aus der Gruppe ausgewählt worden ist, die ein Dichlormethan, ein Chloroform, ein Ethylacetat, ein Methyl-ethyl-keton und ein gemischtes Lösungsmittel davon umfasst, und
wobei das zweite Lösungsmittel aus das Schritt (a) mindestens eines ist, das aus der Gruppe ausgewählt worden ist, die ein Methylalkohol, ein Ethylalkohol, ein Aceton, ein Acetonitril, ein Dimethylsulfoxid, ein Dimethylformamid, ein N-Methylpyrrolidon, eine Essigsäure und eine Mischung davon umfasst.

6. Verfahren zur Herstellung eines kollagenpeptidhaltigen Polycaprolacton-Mikrokügelchen nach Anspruch 3, wobei das Tensid aus dem Schritt (b) mindestens eines ist, das aus der Gruppe ausgewählt worden ist, die eine Methylcellulose, ein Polyvinylpyrrolidon, eine Carboxymethylcellulose, ein Lecithin, eine Gelatine, ein Polyvinylalkohol, ein Polyoxyethylensorbitanfettsäureester, Polyoxyethylen-Rizinusöl-Derivate und Mischungen davon umfasst.

7. Verfahren zur Herstellung eines kollagenpeptidhaltigen Polycaprolacton-Mikrokügelchen nach Anspruch 3 oder 6, wobei das Tensid aus dem Schritt (b) in einer Menge von 0,01 Gewichtsprozent bis 20 Gewichtsprozent, bezogen auf das Gesamtvolumen der das Tensid enthaltenden wässrigen Lösung, enthalten ist.

8. Polycaprolacton-Mikrokügelchen-Füllstoff, der bezogen auf insgesamt 100 Gewichtsprozent des Polycaprolacton-Mikrokügelchen-Füllstoffs 2 bis 50 Gewichtsprozent eines kollagenpeptidhaltigen Polycaprolacton-Mikrokügelchen, das 0,01 bis 7 Gewichtsprozent des Kollagenpeptids, das in vivo eine kollagenregenerationsfördernde Wirkung zeigt, und eine durchschnittliche Partikelgröße von 10 bis 100 $\mu$m aufweist, 0,1 bis 5 Gewichtsprozent eines gelösten Stoffes, 0 bis 48 Gewichtsprozent eines Gleitmittels, und 15 bis 97,9 Gewichtsprozent eines pharmazeutisch verträglichen wässrigen Trägers, aufweist,

wobei das Polycaprolacton eine inhärente Viskosität von 0,16 bis 1,90 dL/g aufweist,
wobei das Mikrokügelchen einen Span-Wert von 1,0 oder weniger aufweist und wobei der Span-Wert nach der folgenden Formel berechnet wird:

$$(\text{Span-Wert}) = (Dv0,9-Dv0,1)/Dv0,5,$$

wobei in der Formel das $Dv0,1$ eine Partikelgröße bedeutet, die 10 % des Volumenprozentsatzes in der Partikelgrößenverteilungskurve des Mikrokügelchen entspricht, das $Dv0,5$ eine Partikelgröße bedeutet, die 50 % des Volumenanteils in der Partikelgrößenverteilungskurve des Mikrokügelchen entspricht, und das $Dv0,9$ eine Partikelgröße bedeutet, die 90 % des Volumenanteils in der Partikelgrößenverteilungskurve des Mikrokügelchen entspricht.

9. Polycaprolacton-Mikrokügelchen-Füllstoff nach Anspruch 8, wobei das Kollagenpeptid mindestens eines ist, das aus der Gruppe ausgewählt worden ist, die ein KTTKS, ein GHK, ein AHK und deren Derivaten umfasst.

10. Polycaprolacton-Mikrokügelchen-Füllstoff nach Anspruch 8, wobei der gelöste Stoff mindestens einer ist, der aus der Gruppe ausgewählt worden ist, die eine Carboxymethylcellulose (CMC), eine Hydroxypropylmethylcellulose (HPMC), eine Hyaluronsäure, ein Lidocain, ein Polydeoxyribonukleotid (PDRN), ein Polynukleotid (PN) und eine Mischung davon umfasst.

11. Polycaprolacton-Mikrokügelchen-Füllstoff nach Anspruch 8, wobei das Gleitmittel ein Glycerin ist.

12. Polycaprolacton-Mikrokügelchen-Füllstoff nach Anspruch 8, wobei der pharmazeutisch verträgliche wässrige Träger destilliertes Wasser, eine physiologische Kochsalzlösung oder ein Phosphatpuffer ist.

13. Polycaprolacton-Mikrokügelchen-Füllstoff nach Anspruch 8, wobei der Füllstoff zur Verbesserung von Falten, zur Verbesserung von Weichgewebe oder zur Volumenvergrößerung oder zur Korrektur von Konturen dient.

14. Polycaprolacton-Mikrokügelchen-Füllstoff nach Anspruch 8 oder 13, wobei der Füllstoff für eine Injektion bestimmt ist.

15. Fertigspritze, die mit dem Polycaprolacton-Mikrokügelchen-Füllstoff nach einem der Ansprüche 8 bis 14 gefüllt ist.

**Revendications**

1. Microsphère de polycaprolactone contenant un peptide de collagène comprenant de 0,01 à 7 % en poids de peptide de collagène qui présente un effet promoteur de régénération de collagène *in vivo* sur la base d'un total de 100 % en poids de la microsphère de polycaprolactone contenant un peptide de collagène et ayant une taille moyenne de particules de 10 à 100 $\mu$m, dans laquelle la polycaprolactone a une viscosité inhérente de 0,16 à 1,90 dL/g ;

dans laquelle la microsphère a une valeur de span de 1,0 ou moins, et dans laquelle la valeur de span est calculée par la formule suivante :

$$\text{(valeur de span)} = (Dv0,9 - Dv0,1)/Dv0,5$$

dans laquelle, dans ladite formule, Dv0,1 correspond à une taille de particules correspondant à 10 % de la répartition volumique en % sur la courbe de distribution de taille de particules de la microsphère, Dv0,5 correspond à une taille de particules correspondant à 50 % de la répartition volumique en % sur la courbe de distribution de taille de particules de la microsphère, et Dv0,9 correspond à une taille de particules correspondant à 90 % de la répartition volumique en % sur la courbe de distribution de taille de particules de la microsphère.

2. Microsphère de polycaprolactone contenant un peptide de collagène selon la revendication 1, dans laquelle le peptide de collagène est au moins un élément choisi parmi le groupe constitué de KTTKS, GHK, AHK, et de dérivés de ceux-ci.

3. Procédé de préparation d'une microsphère de polycaprolactone contenant un peptide de collagène, le procédé comprenant les étapes suivantes :

(a) la préparation d'une solution unique en dissolvant une polycaprolactone dans un premier solvant et en dissolvant un peptide de collagène qui présente un effet promoteur de régénération de collagène *in vivo* dans un deuxième solvant de façon à préparer chaque solution, puis le mélange de manière homogène des deux solutions afin de préparer une solution unique ;
(b) la préparation d'une émulsion en mélangeant la solution unique obtenue à l'étape (a) avec une solution aqueuse (phase continue) contenant un tensioactif ;
(c) l'extraction de solvant organique de la solution unique utilisée comme phase dispersée dans une phase continue au sein de l'émulsion préparée à l'étape (b) et l'évaporation dudit solvant organique de la surface de la phase continue afin de former une microsphère ; et
(d) la récupération de la microsphère à partir de la phase continue de l'étape (c) pour préparer une microsphère de polycaprolactone,

lorsque l'émulsion est formée à l'aide d'un agitateur à grande vitesse à l'étape (b), le procédé comprend un processus de tamisage supplémentaire entre les étapes (c) et (d) afin d'obtenir une microsphère uniforme ;
dans lequel la polycaprolactone a une viscosité inhérente de 0,16 à 1,90 dL/g ;
dans lequel la microsphère comprend 0,01 à 7 % en poids de peptide de collagène sur la base d'un total de 100 % en poids de la microsphère de polycaprolactone contenant un peptide de collagène et a une taille moyenne de particules de 10 à 100 $\mu$m ;
dans lequel la microsphère a une valeur de span de 1,0 ou moins, et dans lequel la valeur de span est calculée par la formule suivante :

$$\text{(valeur de span)} = (Dv0,9 - Dv0,1)/Dv0,5$$

dans lequel, dans ladite formule, Dv0,1 correspond à une taille de particules correspondant à 10 % de la répartition volumique en % sur la courbe de distribution de taille de particules de la microsphère, Dv0,5 correspond à une taille de particules correspondant à 50 % de la répartition volumique en % sur la courbe de distribution de taille de particules de la microsphère, et Dv0,9 correspond à une taille de particules correspondant à 90 % de la répartition volumique en % sur la courbe de distribution de taille de particules de la microsphère.

4. Procédé de préparation d'une microsphère de polycaprolactone contenant un peptide de collagène selon la revendication 3, dans lequel le peptide de collagène est au moins un élément choisi parmi le groupe constitué de KTTKS, GHK, AHK, et de dérivés de ceux-ci.

5. Procédé de préparation d'une microsphère de polycaprolactone contenant un peptide de collagène selon la revendication 3,

dans lequel le premier solvant de l'étape (a) est au moins un élément choisi parmi le groupe constitué de dichlorométhane, de chloroforme, d'acétate d'éthyle, de méthyléthylcétone et d'un solvant issu d'un mélange de ceux-ci et

dans lequel le deuxième solvant de l'étape (a) est au moins un élément choisi parmi le groupe constitué d'alcool méthylique, d'alcool éthylique, d'acétone, d'acétonitrile, de diméthylsulfoxyde, de diméthylformamide, de N-méthylpyrrolidone, d'acide acétique et d'un mélange de ceux-ci.

6. Procédé de préparation d'une microsphère de polycaprolactone contenant un peptide de collagène selon la revendication 3,
dans lequel le tensioactif de l'étape (b) est au moins un élément choisi parmi le groupe constitué de méthylcellulose, de polyvinylpyrrolidone, de carboxyméthylcellulose, de lécithine, de gélatine, d'alcool polyvinylique, d'ester d'acide gras de polyoxyéthylène sorbitane, de dérivés d'huile de ricin polyoxyéthylénée et de mélanges de ceux-ci.

7. Procédé de préparation d'une microsphère de polycaprolactone contenant un peptide de collagène selon la revendication 3 ou 6,
dans lequel le tensioactif de l'étape (b) est présent en une quantité de 0,01 % p/v à 20 % p/v sur la base du volume total de la solution aqueuse contenant le tensioactif.

8. Produit de comblement à base de microsphères de polycaprolactone comprenant 2 à 50 % en poids d'une microsphère de polycaprolactone contenant un peptide de collagène, laquelle microsphère comprend 0,01 à 7 % en poids du peptide de collagène qui présente un effet promoteur de régénération de collagène *in vivo* et a une taille moyenne de particules de 10 à 100 $\mu$m ; 0,1 à 5 % en poids d'un soluté ; 0 à 48 % en poids d'un lubrifiant ; et 15 à 97,9 % en poids d'un vecteur aqueux pharmaceutiquement acceptable, sur la base d'un total de 100 % en poids du produit de comblement à base de microsphères de polycaprolactone,

dans lequel la polycaprolactone a une viscosité inhérente de 0,16 à 1,90 dL/g ;
dans lequel la microsphère a une valeur de span de 1,0 ou moins, et dans lequel la valeur de span est calculée par la formule suivante :

$$\text{(valeur de span)} = (Dv0,9 - Dv0,1)/Dv0,5$$

dans lequel dans ladite formule, Dv0,1 correspond à une taille de particules correspondant à 10 % de la répartition volumique en % sur la courbe de distribution de taille de particules de la microsphère, Dv0,5 correspond à une taille de particules correspondant à 50 % de la répartition volumique en % sur la courbe de distribution de taille de particules de la microsphère, et Dv0,9 correspond à une taille de particules correspondant à 90 % de la répartition volumique en % sur la courbe de distribution de taille de particules de la microsphère.

9. Produit de comblement à base de microsphères de polycaprolactone selon la revendication 8, dans lequel le peptide de collagène est au moins un élément choisi parmi le groupe constitué de KTTKS, GHK, AHK, et de dérivés de ceux-ci.

10. Produit de comblement à base de microsphères de polycaprolactone selon la revendication 8, dans lequel le soluté est au moins un élément choisi parmi le groupe constitué de carboxyméthylcellulose (CMC), d'hydroxypropylméthylcellulose (HPMC), d'acide hyaluronique, de lidocaïne, de polydésoxyribonucléotide (PDRN), de polynucléotide (PN) et d'un mélange de ceux-ci.

11. Produit de comblement à base de microsphères de polycaprolactone selon la revendication 8, dans lequel le lubrifiant est la glycérine.

12. Produit de comblement à base de microsphères de polycaprolactone selon la revendication 8, dans lequel le vecteur aqueux pharmaceutiquement acceptable est de l'eau distillée, un soluté physiologique salin ou un tampon phosphate.

13. Produit de comblement à base de microsphères de polycaprolactone selon la revendication 8,
dans lequel le produit de comblement est destiné à améliorer les rides, à réparer les tissus mous ou en augmenter le volume, ou à corriger les contours.

**14.** Produit de comblement à base de microsphères de polycaprolactone selon la revendication 8 ou 13, dans lequel le produit de comblement est destiné à une injection.

**15.** Seringue préremplie remplie du produit de comblement à base de microsphères de polycaprolactone selon l'une quelconque des revendications 8 à 14.

[FIG. 1a]

Example 1-1

[FIG. 1b]

Example 3-1

[FIG. 1c]

Example 3-2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005097061 A1
- US 6531160 B2
- WO 0015188 A1
- EP 2803351 A1